Europäisches Patentamt

European Patent Office

Office européen des brevets

(11) Publication number: **0 212 385**
**A2**

(19)

(12) # EUROPEAN PATENT APPLICATION

(21) Application number: **86110620.1**

(22) Date of filing: **31.07.86**

(51) Int. Cl.⁴: **C 12 N 15/00,** C 12 N 1/20,
**C 12 P 21/02**

(30) Priority: **05.08.85 US 762245**

(43) Date of publication of application: **04.03.87**
**Bulletin 87/10**

(84) Designated Contracting States: **CH DE FR GB LI SE**

(71) Applicant: **CORNELL RESEARCH FOUNDATION, INC.,**
**East Hill Plaza, Ithaca, N.Y. 14850 (US)**

(72) Inventor: **Nasrallah, June B., Prof., 109 Brook Drive,**
**Ithaca N.Y. 14850 (US)**
Inventor: **Nasrallah, Mikhail E., 109 Brook Drive, Ithaca**
**N.Y. 14850 (US)**
Inventor: **Goldberg, Michael L., Prof., 227 Renwick Drive,**
**Ithaca N.Y. 14850 (US)**

(74) Representative: **Kraus, Walter, Dr., Patentanwälte**
**Kraus, Weisert & Partner Thomas-Wimmer-Ring 15,**
**D-8000 München 22 (DE)**

(54) **A CDNA clone encoding an S-locus specific glycoprotein.**

(57) A DNA having a base sequence coding for an S-locus gly-
coprotein from self-incompatible plants has been cloned,
novel recombinant DNA transfer vectors containing the cloned
DNA have been constructed, and novel microorganisms trans-
formed by the recombinant transfer vectors have been ob-
tained. A chimeric gene for an S-locus specific glycoprotein
has been constructed and a fusion protein can be expressed
from the chimeric gene. A method of cloning a cDNA se-
quence for S-locus specific glycoprotein from self-incompati-
ble plants is described.

- 1 -

# A cDNA CLONE ENCODING AN S-LOCUS
# SPECIFIC GLYCOPROTEIN

This patent was funded in part by National Science Foundation Grant PCM-8118035.

## FIELD OF THE INVENTION

The present invention relates generally to a novel cloned DNA fragment, and, in particular, the present invention relates to a cloned DNA fragment having a base sequence coding for an S-locus specific glycoprotein from a self-incompatible plant and to a method for obtaining the cloned DNA. The cloned DNA is useful for hybrid seed production.

## BACKGROUND OF THE INVENTION

In over 3000 species representing very diverse angiosperm families, self-fertilization is blocked by a mechanism termed "self-incompatibility" (East, E.M. Proc. Am. Phil. Soc. 82, 449-518 (1940)). Following self-pollination, interaction between the stigma or style and pollen elicits a defined morphological response preventing normal pollen tube growth; fertilization is prevented because these pollen tubes are unable to penetrate the full length of the stigma and style. The specificity of this interaction is determined by one or more genetic loci, and requires the identity of alleles carried by the male and female parents.

Incompatibility systems have been divided into two major groups, the gametophytic systems in which the phenotype of a pollen grain is determined by the haploid pollen genotype, and the sporophytic systems in which pollen phenotype is determined by the genotype of the diploid parental plant. In the sporophytic mode of incompatibility, exemplified by the crucifer Brassica, the self-incompatible reaction is localized at the stigma surface, and occurs within minutes after the initial contact between the pollen and the papillar cells on the outer surface of the stigma. In this genus, self-incompatibility is under the control of a single genetic locus, the S-locus, which is highly polymorphic in that 50 or more alleles have been identified in natural populations (Thompson, K.F. et al, Heredity 21:345-362 (1966); Ockendon, D.J., Heredity 33:159-171 (1974)).

Perhaps the most experimentally tractable route towards a molecular analysis of the genetic control of incompatibility is suggested by the detection of antigens specific to various S-locus alleles in stigma homogenates from different Brassica strains (Nasrallah, M.E. et al, Heredity 22, 519-527 (1967)). These antigens have been shown to correspond to glycoproteins which may be resolved in various electrophoretic systems (Nasrallah, M.E. et al, Heredity, 25, 23-27 (1970); Nasrallah, M.E. et al, Genet. Rs. 20, 151-160 (1972); Nishio, T. et al, Heredity 38, 391-396 (1977); Nasrallah, M.E. et al, Experientia 40, 279-281 (1984)). Several lines of evidence suggest that these glycoproteins play an important role in incompatibility: (1) The mobilities of these molecules vary in stigma extracts derived from Brassica strains with different S-locus alleles. (2) These molecules are found exclusively in the stigma and specifically in the surface papillar cells of the stigma, the site of the initial contact between the pollen and stigma. (3) An increased rate of synthesis of these S-locus specific glycoproteins ("SLSGs") in the developing stigma correlates well with the onset of the incompatibility reaction in the stigma (Nasrallah, J.B. et al, Experientia 40, 279-281 (1984)). (4) Most important, the inheritance of the various forms of SLSG correlates perfectly with the segregation of the S alleles in the $F_1$ and $F_2$ generations, indicating that the gene responsible for this polymorphism must be genetically extremely closely linked to the S-locus (Nasrallah, M.E. Genet. Rs. 20, 151-160 (1972); Hinata, K. et al, Heredity 41, 93-100 (1978)).

Isolation of a cDNA clone encoding an S-locus specific glycoprotein is of great interest because such a clone would allow for identification of self-incompability genes in breeding

0212385

- 4 -

stock; identification of stable S-alleles with strong promoters, as well as introduction of self-incompatibility genes into appropriate plant strains by transformation. Additionally, transformed strains carrying the S-alleles can be interplanted with strains carrying different S-alleles so the seed that is harvested is entirely hybrid. Such hybrid varieties are desired because higher yields are produced. Further, such strains are likely to be better suited for introduction of disease resistance and to have better adaptability to extreme environmental growth conditions. For example, in ornamental crops, self-incompatible varieties have a longer shelf life and flower over a longer season.

<u>SUMMARY OF THE INVENTION</u>

Accordingly, one object of the present invention is to facilitate hybrid seed production by more efficient transfer of stable S-alleles into breeding stocks.

A further object of the present invention is the breakdown of self-incompatibility in fruit trees, resulting in higher yields and better fruit set in the home garden where only one fruit tree is desired.

An even further object of the invention is to replace maintenance of self-incompatible inbred lines by hand pollination.

Still another object of the present invention is to promote interspecific hybridization between self-incompatible and self-compatible species to promote a wider gene pool for plant improvement.

These and other objects have been attained by providing a substantially pure DNA base sequence coding for an S-locus specific glycoprotein from a self-incompatible plant and any fragment of the DNA base sequence which is unique to the DNA base sequence and any functional equivalent of the DNA base sequence or fragment thereof.

The present invention also provides a substantially pure cDNA sequence corresponding to the mRNA sequence coding for an S-locus specific glycoprotein from a self-incompatible plant; a recombinant DNA transfer vector containing a DNA base sequence coding for an S-locus specific glycoprotein from a self-incompatible plant or containing a cDNA base sequence corresponding to a mRNA sequence coding for an S-locus specific glycoprotein from self-incompatible plants; a microorganism transformed by either of the above-mentioned DNA transfer vectors; a chimeric gene comprising a gene or part thereof for an S-locus specific

0212385

- 6 -

glycoprotein and another gene or part thereof; a fusion protein comprising an S-locus specific glycoprotein or part thereof from self-incompatible plants and another protein or part thereof; and a fusion protein that is capable of reacting with antiserum to S-locus specific glycoprotein from self-incompatible plants.

This invention further provides a method for cloning a cDNA sequence coding for at least a portion of an S-locus specific glycoprotein from the self-incompatible plant family Brassica.

## BRIEF DESCRIPTION OF THE FIGURES

Figure 1 is a restriction map and DNA sequencing strategy of the Brassica cDNA insert of pBOS5. The restriction map was constructed by gel electrophoretic analysis of single and double enzyme digests of pBOS5. The thick line represents the coding region for the S-locus specific glycoprotein, and the thin line the 3'-noncoding region of the cDNA sequence. Arrows below the map indicate the direction and extent of sequences subcloned into M13 vectors and read from each restriction site.

Figure 2 is a nucleotide and deduced amino acid sequence of the cDNA insert of pBOS5. The restriction sites given in Figure 1 are indicated. The box outlines the polyadenylation signal sequence, 5'-AATAA-3'.

Figure 3 describes subcloning of the pBOS5 cDNA insert into the pWR590 series of expression vectors in which:

(A) is the generation of the pBOS5 L and S fragments by Bam H1-XhoI digestion. Arrows indicate the resulting direction of transcription in the chimeric plasmid;

(B) is the map of pWR590. Arrows indicate the direction of transcription. Lacz' denotes a shortened lacz gene encoding about 590 amino acids. The expanded region following the lacz' gene contains the polylinker sequence; and

(C) is the nucleotide sequences of the expression vectors at the fusion junctions, and the expected translational products. "nucs" stands for nucleotides, "aas" stands for amino acids.


## DETAILED DESCRIPTION OF THE INVENTION

### Method of Obtaining Clone

The strategy for isolating SLSG-encoding DNA sequences was predicated on the discovery that, in some self-incompatible strains such as Brassica strains, 1-5% of the protein synthesis in the self-incompatible bud stigma is devoted to the synthesis of this macromolecule (Nasrallah, J.B. et al, Experientia 40, 279-281 (1984)). Thus, it seemed likely that a high proportion of clones in a cDNA library constructed with polyA+ RNA from mature, incompatible stigmas would contain SLSG sequences. It had also been previously observed that SLSG molecules appear in the stigma but not in leaf or seedling tissue (Nasrallah, J.B. et al, Experientia 40, 279-281 (1984)). The present inventors, therefore, hypothesized that SLSG cDNA clones would be enriched in that fraction of clones in such a library which would hybridize strongly to radioactive cDNA corresponding to mature stigma mRNA, but not to labeled leaf or seedling-specific cDNA.

A cDNA library according to the present invention can be constructed by preparing cDNA from polyA+ RNA from incompatible stigmas at a time when maximal synthesis of SLSG occurs by priming with oligo(dT)$_{12-18}$ according to a previously described procedure (Maniatis, T. et al, Molecular Cloning: A Laboratory Manual (1982)).

As plant sources for obtaining incompatible stigmas, natural populations and plants resulting from breeding programs at universities, experimental stations and seed companies can be used. Examples of suitable plants from natural populations include Lycopersicon peruvianum and solanum. Examples of suitable plants from breeding programs include vegetable crops such as potatoes, tomatoes, cole crops, beets; ornamental crops such as petunia, nicotiana; cereals and grasses such as rye; stone fruits such as cherries, plums and apples; forage crops such as cloves, kales and mustards; oil seeds such as grape seeds, especially <u>Brassica</u> <u>napus</u>. Especially preferred are self-incompatible <u>Brassica</u> <u>oleracea</u> plants homozygous for various S-alleles such as $S_6$, $S_{13}$, and $S_{14}$ and 47 other alleles known to date (Thompson, K.F. et al, <u>Heredity</u>, <u>21</u>: 345-362 (1966); Ockendon, D.J., <u>Heredity</u>, <u>33</u>, 159-171 (1974).

In order to achieve homozygosity of <u>Brassica</u> <u>oleracea</u>, mature pollen can be applied to immature and, therefore, self-compatible stigmas of the same plant.

Examples of suitable S-homozygotes include all stable S-homozygotes among the fifty known ones. Preferred homozygotes are the $S_6$ and $S_{14}$ homozygotes from <u>Brassica</u> <u>oleracea</u>.

PolyA+ RNA can be isolated by known methods such as oligo(dT) chromatography (Aviv, H. et al, <u>Proc.</u> <u>Natl</u> <u>Acad.</u> <u>Sci.</u> <u>USA</u> <u>69</u>, 1408 (1972)).

The cDNA prepared from polyA+ RNA from incompatible stigmas is treated with $S_1$ nuclease and subsequently repaired with the Klenow fragment of <u>E.</u> <u>coli</u> DNA polymerase to generate blunt-ended cDNA. (Maniatis, T. et al, <u>Molecular</u> <u>Cloning</u>: <u>A</u> <u>Laboratory</u> <u>Manual</u> (1982)). The blunt-ended cDNA can be ligated to restriction endonuclease linkers and digested to completion with the restriction endonuclease. (Maniatis, T. et al, <u>Molecular</u> <u>Cloning</u>: <u>A</u> <u>Laboratory</u> <u>Manual</u> (1982)). Fragments of cDNA larger than 500 base pairs ("bp") can be isolated by known means including electrophoresis on an agarose gel, and cloned into the appropriate restriction site of a plasmid. The plasmids are then used to transform cells by any known method such as the method of Hanahan, D., J. Mol. Biol. 166, 557-580 (1983).

Suitable restriction endonuclease linkers include any linkers containing restriction sites that correspond to unique restriction sites in the plasmid.

Examples include Eco $R_1$ linkers; Hin D linkers and Bam H1 linkers and especially preferred restriction endonuclease linkers for Brassica oleracea are Bam H1 linkers.

Suitable plasmids include any plasmids containing unique restriction sites for insertion of foreign DNA. Examples include pBR322 and pKC7. The plasmid pBR322 is preferred.

Suitable microorganism host cells include any cells that will support replication of the recombinant plasmids. Examples include E. coli HB101; JM101 and SF8 strains. E. coli SF8 are preferred.

Stigma and seedling cDNA probes can be prepared by known methods such as random primed reverse transcription of polyA+ RNA (Taylor, J.M. et al, Biochim. Biophys., Acta 442, 324 (1976). Stigma probes are prepared from polyA+ RNA purified from self-incompatible stigmas, and seedling probes are prepared from polyA+ RNA purified from etiolated seedlings.

Recombinant clones can be screened by any known method including the colony hybridization method of Grunstein & Hogness (Grunstein, M. et al, <u>Proc.</u> <u>Natl</u> <u>Acad.</u> <u>Sci.</u> <u>USA</u> <u>72</u>, 3961 (1975)).

Once the cDNA library is constructed, independent clones can be picked randomly from the library, and DNA from these colonies characterized by known methods such as transferring to each of two nitrocellulose filters, and hybridizing with the appropriate probes. For <u>Brassica</u>, colonies found to hybridize strongly with stigma-derived cDNA probe but to not react with seedling cDNA probe can be selected for further use. One such plasmid which has stigma-derived DNA homologous to that from <u>Brassica</u> <u>oleracea</u> is pBOS5 having ATCC accession No. 40191.

Once the colonies are selected, plasmid DNA can be prepared from each of the colonies, and digests with the appropriate restriction endonuclease can be performed such that the cDNA inserts can be separated from the plasmid vector by gel electrophoresis.

The amplification and isolation of plasmid DNAs can be carried out using standard procedures (Maniatis, T. et al, <u>Molecular</u> <u>Cloning</u> <u>a</u> <u>Laboratory</u> (1982)). Inserts can be separated from the plasmid by digestion with the appropriate restriction endonuclease followed by agarose gel electrophoresis and electroelution (McDonald, M.W. et al, J. Mol. Biol. 110, 119 (1977)).

Cross-hydridization experiments can then be performed on these digests to determine if the DNAs from the plasmids are homologous to the DNA from the original plant source.

Plasmids containing homologous DNA are thus determined.


Determination of Nucleotide Sequence of the cDNA Insert in pBOS5

In order to determine the nucleotide sequence of the cDNA insert, restriction endonuclease-generated fragments of the plasmid insert can be subcloned into appropriate vectors and the resulting single-stranded DNAs subjected to sequence analysis by known techniques such as the Sanger technique (1979). Sanger, F. et al, J. Molec. Biol., 143 161-178 (1980).

Appropriate vectors for subcloning are readily available and readily known to the those skilled in the art. Especially preferred vectors for the pBOS5 insert are M13 vectors mp10 and mp11 (Messing, J. et al, Gene 19, 269-2766 (1982)).

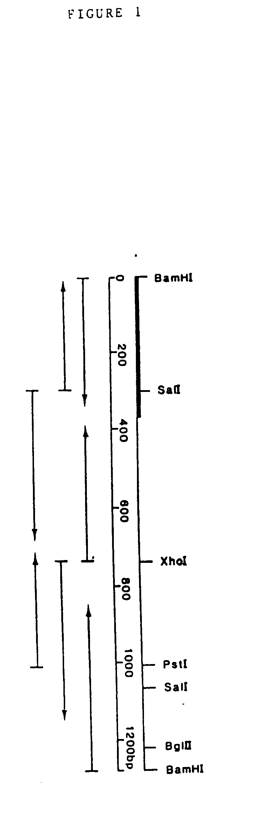

The restriction map and DNA sequencing strategy of the Brassica cDNA insert of pBOS5 is shown in Figure 1.

The restriction map was constructed by gel electrophoretic analysis of various single and double enzyme digests of pBOS5. The thick line represents the coding region, and the thin line

- 13 -

represents the 3'-noncoding region of the cDNA sequence. Arrows below the map indicate the direction and extent of sequences subcloned into M13 vectors and read from each restriction site.

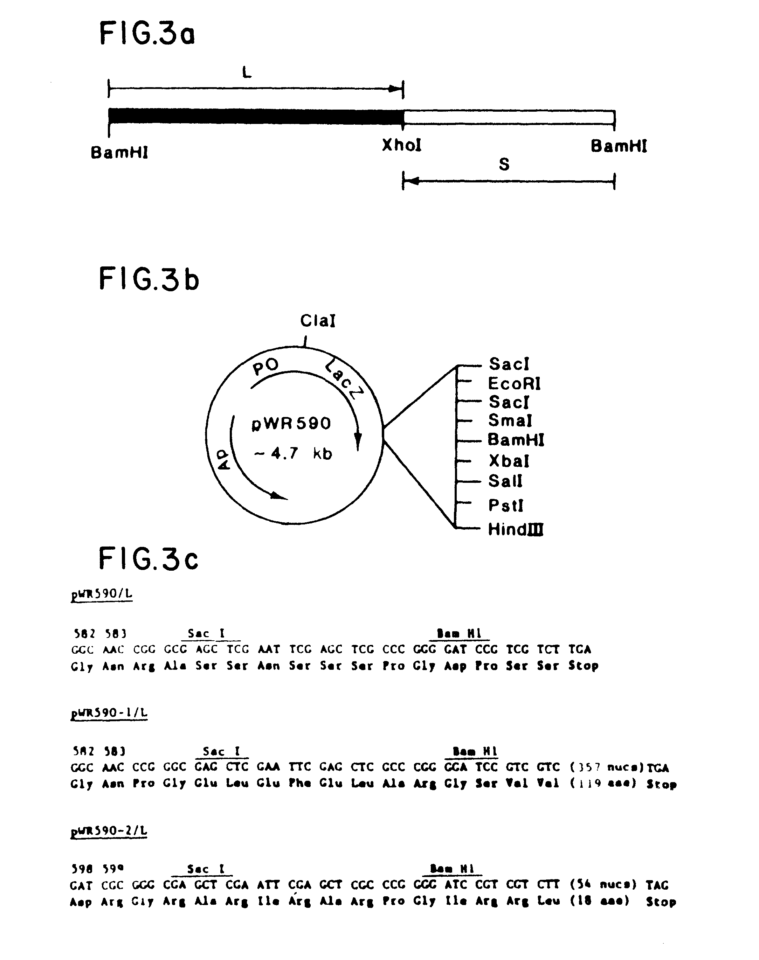

The results of the nucleotide sequence determination on a pBOS5 BamH1 insertion subcloned into M13mp10 and M13mp11 are presented in Figure 2. Although five of the six possible reading frames are blocked by multiple nonsense codons throughout the entire 1283 base pair ("bp") insert, the remaining reading frame translates into a polypeptide 123 amino acids in length as shown in Figure 2. The nucleotide sequence AATAAA, which has been implicated as a signal for the polyadenylation of mRNA (Proudfoodt, N.J. et al, Nature 263, 211-214 (1976)), is located downstream of the SLSG coding region, 11-16 bp from the end of the cDNA insert presumed to have been derived from the 3' end of the mRNA. The cDNA sequence does not contain a poly(A) tail, which is usually 11-30 bp from the polyadenylation signal. An explanation for the absence of a poly(A) tract is provided by the finding that the BamH1 sequences flanking both sides of the cDNA insert do not resemble the BamH1 linker sequence used in the construction of the cDNA clone. While not desiring to be bound, it is presumed that a BamH1 recognition site is located in the full-length cDNA sequence between the polyadenylation signal and the poly(A) tail of the messenger, such that this BamH1 site would be inserted into the pBR322 vector in place of the end provided by the linker sequence.

## Construction of Expression Plasmids

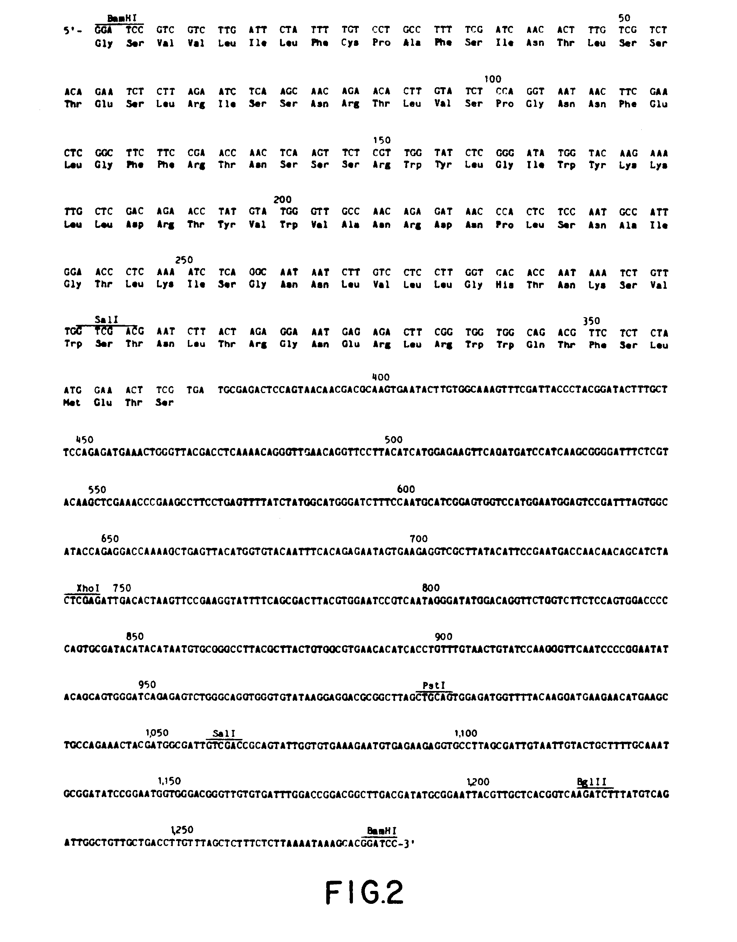

Appropriate expression vectors are vectors that contain sequences of DNA that are required for the transcription of cloned copies of genes and for the translation of their mRNAs in E. coli. Examples include gt11, puc8, puc9, pWR590, pWR590-1, pWR590-2. Preferred vectors for the PBOS5 insert are the three expression vectors pWR590, pWR590-1, and pWR590-2 which have recently been described (Guo, L.H. et al, Gene 29, 251-254 (1984)). In these vectors foreign DNA can be inserted into a polylinker region such that these exogenous sequences can be translated by E. coli cells into a fusion protein, the first 590 amino acids of which are supplied by a truncated E. coli β-galactosidase gene (Guo, L.H. et al, Gene 29, 251-254 (1984) and Figure 5a). By employing all three vectors, a fragment of foreign DNA may be joined to the β-galactosidase gene in all three possible translational reading frames.

The presumptive coding region of the cDNA insert from Brassica oleracea is entirely contained within a fragment of DNA (fragment L in Figure 3B) demarcated by recognition sites for the enzymes BamH1 (at positions 1-6) and Xho I (nucleotides 740-745).

Thus, as illustrated in Figure 3C, fragment L from pBOS5 would theoretically encode a fusion protein containing determinants encoded by the long open reading frame predicted by the DNA sequence data only when inserted into the vector pWR590-1. When placed in either of the remaining two vectors, pWR590 and pWR590-2, fragment L would not be in the correct reading frame with respect to the β-galactosidase gene, and thus would not direct the synthesis of a fusion protein substantially specified by the <u>Brassica</u> DNA.

Six expression plasmids can thus be constructed, as, for example, the six expression plasmids constructed from the L and S strands of <u>Brassica</u>. The resulting six constructs can be individually incorporated into appropriate cells by transformation, and the plasmids in several colonies emerging from each transformation can be examined by digestion with appropriate restriction endonucleases.

Suitable cells for transformation of the expression plasmid include any strains that allow its replication and translation. Examples include <u>E. coli</u> strains HB101, JM101 and SF8. Especially preferred are <u>E. coli</u> SF8.

One colony from each experiment was selected which yielded the expected pattern of fragments. From <u>Brassica</u>, the expected pattern is of two fragments, one corresponding in size to either fragment L or to fragment S, and the other fragment identical in length to the vector.

Extracts can be prepared from cultures of the selected transformants and fractionated by suitable means such as SDS polyacrylamide gel electrophoresis. The proteins analyzed in this manner can be electrophoretically transferred to nitrocellulose filters and probed with antibodies to SLGS by known methods. The plasmid containing fragment L linked to the vector that directs the synthesis of SLGS-related polypeptides can be identified by a positive reaction with SLSG antiserum or by any other suitable method. For Brassica, the size of the prominent band should be that expected for a fusion protein consisting of the amino acids from the expression plasmid and the polylinker region and 123 amino acids encoded by the Brassica cDNA clone.

As already mentioned, the present invention has numerous uses. One such use is to identify S-genes in breeding stock by, for example, Southern blot analysis of genomic DNA. Another use is to identify stable S-alleles with strong promoters by, for example, isoelectric focusing or transformation. Additionally, transformed strains carrying the S-alleles can be interplanted with strains carrying different S-alleles so the seed that is harvested is entirely hybrid. Further uses include: facilitating hybrid seed production by more efficient transfer of stable S-alleles into breeding stocks by, for example, transformation; breaking down self-incompatibility in fruit trees by, for example, _in vitro_ mutagenesis of S-genes, or by chemical and immunochemical blocking of self-incompatibility inbred lines by, for example, transformation or _in vitro_ mutagenesis.

The present invention will now be described by reference to specific examples which are not meant to be limiting.

## Example 1

## Preparation of Clone Containing Brassica Oleracea cDNA

_Brassica oleracea_ self-incompatible plants homozygous for the $S_6$, $S_{13}$, and $S_{14}$ alleles maintained at the State University of New York, Cortland, were used for the following experiment.

Homozygosity was achieved by applying mature pollen to immature and, therefore, self-compatible, stigmas of the same plant.

_Brassica oleracea_ cDNA was prepared from polyA+ RNA from stigmas at one day prior to anthesis by priming with oligo(dT)$_{12-18}$ according to a previously described procedure (Maniatis, T. et al, _Molecular Cloning a Laboratory Manual_ (1982)).

After treatment with $S_1$ nuclease (Bethesda Research Laboratories, BRL) and subsequent repair with the Klenow fragment of E. coli DNA polymerase I (BRL) (Maniatis, T. et al, Molecular Cloning: A Laboratory Manual (1982)), the blunt-ended cDNA was ligated to Bam H1 linkers (BRL) by known methods and digested to completion with Bam H1 by known methods (Maniatis, T. et al, Molecular Cloning: A Laboratory Manual (1982)). cDNA species larger than 500 bp were isolated by electrophoresis on a 1% low-melting agarose gel, in 0.089 molar Tris-base, 0.089 molar boric acid, 0.002 molar ethylenediaminetetraacetic acid (disodium salt), and cloned into the Bam H1 site of the plasmid pBR322 by known methods (Maniatis, T. et al, Molecular Cloning: A Laboratory Manual (1982)). Transformation of E. coli SF8 cells commonly available was performed according to the method of Hanahan (Hanahan, D., J. Mol. Biol. 166, 557-580 (1982)).

Approximately 2,000 independent clones were picked randomly from the cDNA library, and DNA from these colonies was then transferred to each of two nitrocellulose filters, and hybridized with stigma cDNA and seedling cDNA probes.

Stigma and seedling cDNA probes were prepared by random primed reverse transcription of polyA+ RNA (Taylor, J.M. et al, Biochim. Biophys. Acta 442, 324 (1976)). Stigma probes were prepared from polyA+ RNA purified from Brassica self-incompatible stigmas and seeling probes were prepared from polyA+ RNA purified from <u>Brassica</u> etiolated seedlings.

Recombinant clones were screened by the colony hybridization method of Grunstein & Hogness (Grunstein, M. et al, <u>Proc. Natl Acad. Sci. USA</u> <u>72</u>, 3961 (1975)).

Eight colonies were found to hybridize strongly with stigma-derived cDNA probe but not to react with seedling derived cDNA probe.

Plasmid DNA was prepared from each of the eight colonies by known methods (Maniatis, T. et al, <u>Molecular Cloning: A Laboratory Manual</u> (1982)).

The plasmid DNA was digested with restriction endonuclease Bam H1 (Maniatis, T. et al, <u>Molecular Cloning: A Laboratory Manual</u> (1982)).

The cDNA inserts were separated from the plasmid vector by agarose gel electrophoresis and electroelution (McDonald, M.W. et al, <u>J. Mol. Biol.</u> <u>110</u>, 119 (1977)) and were labelled with $^{32}$P by nick-translation (Rigby, P.W.J. et al, <u>J. Mol. Biol.</u> <u>113</u>, 237-251 (1977)).

Cross-hybridization experiments between the various isolated plasmids were then performed by known methods (Maniatis, T. et al, <u>Molecular Cloning: A Laboratory Manual</u> (1982)).

The cross-hybridization experiments indicated that all eight recombinant plasmids were homologous to one another. The plasmid containing the largest cDNA insertion was designated pBOS5 and has ATCC accession number 40191.

## Example 2

## Determination of Nucleotide Sequence of Brassica cDNA Insert in pBOS5

Restriction endonuclease-generated fragments of the pBOS5 insert were subcloned into M13 vectors mp10 and mp11 (Messing, J. et al, Gene 19, 269-2766 (1982)) as shown in Figure 1.

DNA sequencing was carried out on the resulting single--stranded DNA by the dideoxynucleotide chain-termination method (Sanger, F. et al, J. Mol. Biol. 143, 161-178 (1980)) using synthetic 17-mer as primer (New England Biolabs). The 17-mer primer had the following sequence:

5'-GTTTTCCCAGTCACGAC-3'.

The results of the nucleotide sequence determination along with a projected amino acid sequence are presented in Figure 2.

## Example 3

### Preparation of Fusion Proteins

Plasmids pWR590, pWR590-1 are a family of expression vectors designed to express genes in each of the three possible translational frames (Guo, L.H. et al, Gene 29, 251-254 (1984)). In order to construct expression plasmids of the Brassica cDNA sequence, the 745 bp (L) and the 538 pb (S) Bam H1-XhoI fragments of pBOS5 were isolated from a 1% low-melting agarose gel having the same composition as described above and were ligated to Bam H1 and Sal I digests of pWR590, pWR590-1 and pWR590-2 by known methods (Maniatis, T. et al, Molecular Cloning: A Laboratory Manual (1982)). The resulting six plasmid constructions designated pWR590-L, pWR590-1L, pWR590-2L, pWR590-S, pWR590-1S and pWRE590-2S were used to transform E. coli SF8 cells.

Transformed E. coli SF8 strains were grown overnight in the presence of 1mM IPTG. Extracts were prepared by sonication in 7 M guanidine hydrochloride (Guo, L.H. et al, Gene 29, 251-254 (1984)), and aliquots equivalent to 500 µl of culture were subjected to electrophoresis on 10% SDS acrylamide gels (Laemnli, U.K., Nature 227, 680-685 (1970)). The separated proteins were electrophoretically transferred to nitrocellulose membranes according to Towbin, H. et al, Proc. Natl Acad. Sci. USA 76, 4350-4354 (1979).

0212385

Immunolabeling of nitrocellulose blots was prepared as described by Symington, J., "Two-dimensional Electrophresis of Proteins", Academic Press (1983). The first stage antiserum was raised in a New Zealand White rabbit by multiple intradermal injections of $S_6$-specific glycoprotein purified by preparative isoelectric focusing in granulated beds. The second stage probe was peroxidase-conjugated protein A.

## Example 4

Genomic DNAs isolated from various _Brassica_ species, i.e., _Brassica oleracea_, _Brassica campestris_, _Brassica napus_, _Brassica juncea_, _Brassica nigra_, and _Brassica carinata_, and from other genera in the same family of Cruciferae (also known as Brassicaceae) such as _Raphanus_, _Lepidium_ and _Histera_ were subjected to Southern analyses (Southern, E. _J. Mol. Biol._, 98, 503-517 (1975)). The nitrocellulose blots of restriction digests of these DNAs were probed with $^{32}$P-labelled pBOS5. In all cases bonds of sequences that hybridize to pBOS5 and that are, therefore, homologous to pBOS5 were detected.

While the invention has been described in detail and with reference to specific embodiments thereof, it will be apparent to one skilled in the art that various changes and modifications can be made therein without departing from the spirit and scope thereof.

WHAT IS CLAIMED IS:

1.    A substantially pure DNA base sequence coding for an S-locus specific glycoprotein from self-incompatible plants and any fragment of said DNA base sequence which is unique to said DNA base sequence and any functional equivalent of said DNA base sequence or fragment thereof.

2.    A substantially pure DNA base sequence of Claim 1, wherein a noncoding complementary strand of the DNA base sequence is as follows:

```
                                                   50
5'- GGA TCC GTC GTC TTG ATT CTA TTT TGT CCT GCC TTT TCG ATC AAC ACT TTG TCG TCT

                                          100
ACA GAA TCT CTT AGA ATC TCA AGC AAC AGA ACA CTT GTA TCT CCA GGT AAT AAC TTC GAA

                                     150
CTC GGC TTC TTC CGA ACC AAC TCA AGT TCT CGT TGG TAT CTC GGG ATA TGG TAC AAG AAA

                           200
TTG CTC GAC AGA ACC TAT GTA TGG GTT GCC AAC AGA GAT AAC CCA CTC TCC AAT GCC ATT

                 250
GGA ACC CTC AAA ATC TCA GGC AAT AAT CTT GTC CTC CTT GGT CAC ACC AAT AAA TCT GTT

                                          350
TGG TCG ACG AAT CTT ACT AGA GGA AAT GAG AGA CTT CGG TGG TGG CAG ACG TTC TCT CTA

                                400
ATC GAA ACT TCG TGA TGCGAGACTCCAGTAACAACGACGCAAGTCAATACTTGTGGCAAAGTTTCGATTACCTACGGATACTTTGCT

  450                                        500
TCCAGAGATGAAACTGGGTTACGACCTCAAAACAGGGTTGAACAGGTTCCTTACATCATGGAGAAGTTCAGATGATCCATCAAGCGGGGATTTCTCGT

  550                                        600
ACAAGCTCGAAACCCGAAGCCTTCCTGAGTTTTATCTATGGCATGGGATCTTTCCAATGCATCGGAGTGGTCCATGGAATGGAGTCCGATTTAGTGGC

  650                                        700
AACCAGAGGACCAAAAGCTGAGTTACATGGTGTACAATTTCACAGAGAATAGTGAAGAGGTCGCTTATACATTCCGAATGACCAACAACAGCATCTA

  750                                        800
CTCGAGATTGACACTAAGTTCCGAAGGTATTTTCAGCGACTTACGTGGAATCCGTCAATAGGGATATGGACAGGTTCTGGTCTTCTCCAGTGGACCCC

  850                                        900
CAGTGCGATACATACATAATGTGCGGGGCCTTACGCTTACTGTGGCGTGAACACATCACCTGTTTGTAACTGTATCCAAGGGTTCAATCCCCGGAATAT
```

ACAGCAGTGGGATCAGAGAGTCTGGGCAGGTGGGTGTATAAGGAGGACGCGGCTTAGCTGCAGTGGAGATGGTTTTACAAGGATGAAGAACATGAAGC

TGCCAGAAACTACGATGGCGATTGTCGACCGCAGTATTGGTGTGAAAGAATGTGAGAAGAGGTGCCTTAGCGATTGTAATTGTACTGCTTTTGCAAAT

GCAAATGCGGATATCCGGAATGGTGGGACGGGTTGTGTGATTTGGACCGGACGGCTTGACGATATGCGGAATTACGTTGCTCACGGTCAAGATCTTTATGTCAG

ATTGGCTGTTGCTGACCTTGTTTAGCTCTTTCTCTTAAAATAAAGCACGGATCC-3'

and any fragment of said DNA base sequence which is unique to said DNA base sequence and any functional equivalent of said DNA base sequence or fragment thereof.

3. A substantially pure cDNA base sequence corresponding to mRNA coding for an S-locus specific glycoprotein from self-incompatible plants and any fragment of said cDNA base sequence which is unique to said cDNA base sequence and any functional equivalent of said cDNA sequence or fragment thereof.

4. A substantially pure cDNA base sequence of Claim 3, wherein the cDNA base sequence is as follows:

5'- GGA TCC GTC GTC TTG ATT CTA TTT TGT CCT GCC TTT TCG ATC AAC ACT TTG TCG TCT

ACA GAA TCT CTT AGA ATC TCA AGC AAC AGA ACA CTT GTA TCT CCA GGT AAT AAC TTC GAA

CTC GGC TTC TTC CGA ACC AAC TCA AGT TCT CGT TGG TAT CTC GGG ATA TGG TAC AAG AAA

TTG CTC GAC AGA ACC TAT GTA TGG GTT GCC AAC AGA GAT AAC CCA CTC TCC AAT GCC ATT

GGA ACC CTC AAA ATC TCA GGC AAT AAT CTT GTC CTC CTT GGT CAC ACC AAT AAA TCT GTT

```
                                                   350
TGG TCG ACG AAT CTT ACT AGA GGA AAT GAG AGA CTT CGG TGG TGG CAG ACG TTC TCT CTA


                                  400
ATC GAA ACT TCG TGA TGCGAGACTCCAGTAACAACGACGCAAGTCAATACTTGTGGCAAAGTTTCGATTACCTACGGATACTTTGCT


   450                                       500
TCCAGAGATGAAACTGGGTTACGACCTCAAAACAGGGTTGAACAGGTTCCTTACATCATGGAGAAGTTCAGATGATCCATCAAGCGGGGATTTCTCGT


   550                                       600
ACAAGCTCGAAACCCGAAGCCTTCCTGAGTTTTATCTATGGCATGGGATCTTTCCAATGCATCGGAGTGGTCCATGGAATGGAGTCCGATTTAGTGGC


   650                                       700
AACCAGAGGACCAAAAGCTGAGTTACATGGTGTACAATTTCACAGAGAATAGTGAAGAGGTCGCTTATACATTCCGAATGACCAACAACAGCATCTA


   750                                       800
CTCGAGATTGACACTAAGTTCCGAAGGTATTTTCAGCGACTTACGTGGAATCCGTCAATAGGGATATGGACAGGTTCTGGTCTTCTCCAGTGGACCCC


   850                                       900
CAGTGCGATACATACAATGTGCGGGCCTTACGCTTACTGTGGCGTGAACACATCACCTGTTTGTAACTGTATCCAAGGGTTCAATCCCCGGAATAT


   950
ACAGCAGTGGGATCAGAGAGTCTGGGCAGGTGGGTGTATAAGGAGGACGCGGCTTAGCTGCAGTGGAGATGGTTTTACAAGGATGAAGAACATGAAGC


   1050                                      1100
TGCCAGAAACTACGATGGCGATTGTCGACCGCAGTATTGGTGTGAAAGAATGTGAGAAGAGGTGCCTTAGCGATTGTAATTGTACTGCTTTTGCAAAT


   1150                                      1200
GCAAATGCGGATATCCGGAATGGTGGGACGGGTTGTGTGATTTGGACCGGACGGCTTGACGATATGCGGAATTACGTTGCTCACGGTCAAGATCTTTATGTCAG


   1250
ATTGGCTGTTGCTGACCTTGTTTAGCTCTTTCTCTTAAAATAAAGCACGGATCC-3'
```

and any fragment of said DNA base sequence which is unique to said DNA base sequence and any functional equivalent of said DNA base sequence or fragment thereof.

5.   A recombinant DNA transfer vector containing a DNA base sequence coding for an S-locus specific glycoprotein from a self-incompatible plant and any transfer vector containing any fragment of said DNA base sequence which is unique to said DNA

base sequence and any functional equivalent of said DNA base
sequence or fragment thereof.

6.    A recombinant DNA transfer vector of Claim 5, wherein a
noncoding complementary strand of the DNA base sequence is as
follows:

```
                                                         50
5'- GGA TCC GTC GTC TTG ATT CTA TTT TGT CCT GCC TTT TCG ATC AAC ACT TTG TCG TCT

                                                       100
ACA GAA TCT CTT AGA ATC TCA AGC AAC AGA ACA CTT GTA TCT CCA GGT AAT AAC TTC GAA

                                            150
CTC GGC TTC TTC CGA ACC AAC TCA AGT TCT CGT TGG TAT CTC GGG ATA TGG TAC AAG AAA

                                    200
TTG CTC GAC AGA ACC TAT GTA TGG GTT GCC AAC AGA GAT AAC CCA CTC TCC AAT GCC ATT

                            250
GGA ACC CTC AAA ATC TCA GGC AAT AAT CTT GTC CTC CTT GGT CAC ACC AAT AAA TCT GTT

                                                    350
TGG TCG ACG AAT CTT ACT AGA GGA AAT GAG AGA CTT CGG TGG TGG CAG ACG TTC TCT CTA

                                400
ATC GAA ACT TCG TGA TGCGAGACTCCAGTAACAACGACGCAAGTCAATACTTGTGGCAAAGTTTCGATTACCTACGGATACTTTGCT

    450                                           500
TCCAGAGATGAAACTGGGTTACGACCTCAAAACAGGGTTGAACAGGTTCCTTACATCATGGAGAAGTTCAGATGATCCATCAAGCGGGGATTTCTCGT

    550                                           600
ACAAGCTCGAAACCCGAAGCCTTCCTGAGTTTTATCTATGGCATGGGATCTTTCCAATGCATCGGAGTGGTCCATGGAATGGAGTCCGATTTAGTGGC

    650                                           700
AACCAGAGGACCAAAAGCTGAGTTACATGGTGTACAATTTCACAGAGAATAGTGAAGAGGTCGCTTATACATTCCGAATGACCAACAACAGCATCTA

    750                                           800
CTCGAGATTGACACTAAGTTCCGAAGGTATTTTCAGCGACTTACGTGGAATCCGTCAATAGGGATATGGACAGGTTCTGGTCTTCTCCAGTGGACCCC

    850                                           900
CAGTGCGATACATACATAATGTGCGGGGCCTTACGCTTACTGTGGCGTGAACACATCACCTGTTTGTAACTGTATCCAAGGGTTCAATCCCCGGAATAT
```

950
ACAGCAGTGGGATCAGAGAGTCTGGGCAGGTGGGTGTATAAGGAGGACGCGGCTTAGCTGCAGTGGAGATGGTTTTACAAGGATGAAGAACATGAAGC

1050                                              1100
TGCCAGAAACTACGATGGCGATTGTCGACCGCAGTATTGGTGTGAAAGAATGTGAGAAGAGGTGCCTTAGCGATTGTAATTGTACTGCTTTTGCAAAT

1150                                              1200
GCAAATGCGGATATCCGGAATGGTGGGACGGGTTGTGTGATTTGGACCGGACGGCTTGACGATATGCGGAATTACGTTGCTCACGGTCAAGATCTTTATGTCAG

1250
ATTGGCTGTTGCTGACCTTGTTTAGCTCTTTCTCTTAAAATAAAGCACGGATCC-3'

and any recombinant DNA transfer vector containing any fragment of said DNA base sequence which is unique to said DNA base sequence and any functional equivalent of said DNA base sequence or fragment thereof.

7.    A recombinant DNA transfer vector containing a cDNA base sequence corresponding to the mRNA sequence colding for an S-locus specific glycoprotein form a self-incompatible plant and any transfer vector containing any fragment of said cDNA base sequence which is unique to said cDNA base sequence and any functional sequence or fragment thereof.

8.    A recombinant DNA transfer vector of Claim 7, wherein the cDNA base sequence is as follows:

50
5'- GGA TCC GTC GTC TTG ATT CTA TTT TGT CCT GCC TTT TCG ATC AAC ACT TTG TCG TCT

100
ACA GAA TCT CTT AGA ATC TCA AGC AAC AGA ACA CTT GTA TCT CCA GGT AAT AAC TTC GAA

150
CTC GGC TTC TTC CGA ACC AAC TCA AGT TCT CGT TGG TAT CTC GGG ATA TGG TAC AAG AAA

```
                        200
TTG CTC GAC AGA ACC TAT GTA TGG GTT GCC AAC AGA GAT AAC CCA CTC TCC AAT GCC ATT

            250
GGA ACC CTC AAA ATC TCA GGC AAT AAT CTT GTC CTC CTT GGT CAC ACC AAT AAA TCT GTT

                                            350
TGG TCG ACG AAT CTT ACT AGA GGA AAT GAG AGA CTT CGG TGG TGG CAG ACG TTC TCT CTA

                                400
ATC GAA ACT TCG TGA TGCGAGACTCCAGTAACAACGACGCAAGTCAATACTTGTGGCAAAGTTTCGATTACCTACGGATACTTTGCT

   450                                 500
TCCAGAGATGAAACTGGGTTACGACCTCAAAACAGGGTTGAACAGGTTCCTTACATCATGGAGAAGTTCAGATGATCCATCAAGCGGGGATTTCTCGT

   550                                 600
ACAAGCTCGAAACCCGAAGCCTTCCTGAGTTTTATCTATGGCATGGGATCTTTCCAATGCATCGGAGTGGTCCATGGAATGGAGTCCGATTTAGTGGC

     650                             700
AACCAGAGGACCAAAAGCTGAGTTACATGGTGTACAATTTCACAGAGAATAGTGAAGAGGTCGCTTATACATTCCGAATGACCAACAACAGCATCTA

       750                             800
CTCGAGATTGACACTAAGTTCCGAAGGTATTTTCAGCGACTTACGTGGAATCCGTCAATAGGGATATGGACAGGTTCTGGTCTTCTCCAGTGGACCCC

       850                           900
CAGTGCGATACATACATAATGTGCGGGGCCTTACGCTTACTGTGGCGTGAACACATCACCTGTTTGTAACTGTATCCAAGGGTTCAATCCCCGGAATAT

         950
ACAGCAGTGGGATCAGAGAGTCTGGGCAGGTGGGTGTATAAGGAGGACGCGGCTTAGCTGCAGTGGAGATGGTTTTACAAGGATGAAGAACATGAAGC

         1050                         1100
TGCCAGAAACTACGATGGCGATTGTCGACCGCAGTATTGGTGTGAAAGAATGTGAGAAGAGGTGCCTTAGCGATTGTAATTGTACTGCTTTTGCAAAT

           1150                           1200
GCAAATGCGGATATCCGGAATGGTGGGACGGGTTGTGTGATTTGGACCGGACGGCTTGACGATATGCGGAATTACGTTGCTCACGGTCAAGATCTTTATGTCAG

             1250
ATTGGCTGTTGCTGACCTTGTTTAGCTCTTTCTCTTAAAATAAAGCACGGATCC-3'
```

and any recombinant DNA transfer vector containing any fragment of said DNA base sequence which is unique to said DNA base sequence and any functional equivalent of said DNA base sequence or fragment thereof.

9. A microorganism transformed by a transfer vector containing a cDNA base sequence corresponding to mRNA sequence coding for an S-locus specific glycoprotein from a self-incompatible plant and any transfer vector containing any fragment of said cDNA base sequence which is unique to said cDNA base sequence and any functional equivalent of said cDNA base sequence or fragment thereof.

10. A microorganism of Claim 9, wherein the cDNA base sequence is as follows:

```
                                                        50
5'- GGA TCC GTC GTC TTG ATT CTA TTT TGT CCT GCC TTT TCG ATC AAC ACT TTG TCG TCT

                                                       100
ACA GAA TCT CTT AGA ATC TCA AGC AAC AGA ACA CTT GTA TCT CCA GGT AAT AAC TTC GAA

                                                150
CTC GGC TTC TTC CGA ACC AAC TCA AGT TCT CGT TGG TAT CTC GGG ATA TGG TAC AAG AAA

                                 200
TTG CTC GAC AGA ACC TAT GTA TGG GTT GCC AAC AGA GAT AAC CCA CTC TCC AAT GCC ATT

                        250
GGA ACC CTC AAA ATC TCA GGC AAT AAT CTT GTC CTC CTT GGT CAC ACC AAT AAA TCT GTT

                                                        350
TGG TCG ACG AAT CTT ACT AGA GGA AAT GAG AGA CTT CGG TGG TGG CAG ACG TTC TCT CTA

                                        400
ATC GAA ACT TCG TGA TGCGAGACTCCAGTAACAACGACGCAAGTCAATACTTGTGGCAAAGTTTCGATTACCTACGGATACTTTGCT

   450                                        500
TCCAGAGATGAAACTGGGTTACGACCTCAAAACAGGGTTGAACAGGTTCCTTACATCATGGAGAAGTTCAGATGATCCATCAAGCGGGGATTTCTCGT

   550                                        600
ACAAGCTCGAAACCCGAAGCCTTCCTGAGTTTTATCTATGGCATGGGATCTTTCCAATGCATCGGAGTGGTCCATGGAATGGAGTCCGATTTAGTGGC
```

650                                                    700
AACCAGAGGACCAAAAGCTGAGTTACATGGTGTACAATTTCACAGAGAATAGTGAAGAGGTCGCTTATACATTCCGAATGACCAACAACAGCATCTA

750                                                    800
CTCGAGATTGACACTAAGTTCCGAAGGTATTTTCAGCGACTTACGTGGAATCCGTCAATAGGGATATGGACAGGTTCTGGTCTTCTCCAGTGGACCCC

850                                                    900
CAGTGCGATACATACATAATGTGCGGGCCTTACGCTTACTGTGGCGTGAACACATCACCTGTTTGTAACTGTATCCAAGGGTTCAATCCCCGGAATAT

950
ACAGCAGTGGGATCAGAGAGTCTGGGCAGGTGGGTGTATAAGGAGGACGCGGCTTAGCTGCAGTGGAGATGGTTTTACAAGGATGAAGAACATGAAGC

1050                                                   1100
TGCCAGAAACTACGATGGCGATTGTCGACCGCAGTATTGGTGTGAAAGAATGTGAGAAGAGGTGCCTTAGCGATTGTAATTGTACTGCTTTTGCAAAT

1150                                                   1200
GCAAATGCGGATATCCGGAATGGTGGGACGGGTTGTGTGATTTGGACCGGACGGCTTGACGATATGCGGAATTACGTTGCTCACGGTCAAGATCTTTATGTCAG

1250
ATTGGCTGTTGCTGACCTTGTTTAGCTCTTTCTCTTAAAATAAAGCACGGATCC-3'

and any microorganism transformed by a transfer vector containing any fragment of said DNA base sequence which is unique to said DNA base sequence and any functional equivalent of said DNA base sequence or fragment thereof.

11.   A chimeric gene comprising a first gene, or part thereof for an S-locus specific glycoprotein from a self-incompatible plant and a second gene or part thereof.

12.   A chimeric gene of Claim 11, wherein a noncoding complementary strand of said first gene is as follows:

                                        50
5'- GGA TCC GTC GTC TTG ATT CTA TTT TGT CCT GCC TTT TCG ATC AAC ACT TTG TCG TCT

                                        100
ACA GAA TCT CTT AGA ATC TCA AGC AAC AGA ACA CTT GTA TCT CCA GGT AAT AAC TTC GAA

```
                                        150
CTC GGC TTC TTC CGA ACC AAC TCA AGT TCT CGT TGG TAT CTC GGG ATA TGG TAC AAG AAA

                        200
TTG CTC GAC AGA ACC TAT GTA TGG GTT GCC AAC AGA GAT AAC CCA CTC TCC AAT GCC ATT

            250
GGA ACC CTC AAA ATC TCA GGC AAT AAT CTT GTC CTC CTT GGT CAC ACC AAT AAA TCT GTT

                                                    350
TGG TCG ACG AAT CTT ACT AGA GGA AAT GAG AGA CTT CGG TGG TGG CAG ACG TTC TCT CTA

                                400
ATC GAA ACT TCG TGA TGCGAGACTCCAGTAACAACGACGCAAGTCAATACTTGTGGCAAAGTTTCGATTACCTACGGATACTTTGCT

    450                                          500
TCCAGAGATGAAACTGGGTTACGACCTCAAAACAGGGTTGAACAGGTTCCTTACATCATGGAGAAGTTCAGATGATCCATCAAGCGGGGATTTCTCGT

    550                                          600
ACAAGCTCGAAACCCGAAGCCTTCCTGAGTTTTATCTATGGCATGGGATCTTTCCAATGCATCGGAGTGGTCCATGGAATGGAGTCCGATTTAGTGGC

    650                                          700
AACCAGAGGACCAAAAGCTGAGTTACATGGTGTACAATTTCACAGAGAATAGTGAAGAGGTCGCTTATACATTCCGAATGACCAACAACAGCATCTA

    750                                          800
CTCGAGATTGACACTAAGTTCCGAAGGTATTTTCAGCGACTTACGTGGAATCCGTCAATAGGGATATGGACAGGTTCTGGTCTTCTCCAGTGGACCCC

    850                                          900
CAGTGCGATACATACATAATGTGCGGGGCCTTACGCTTACTGTGGCGTGAACACATCACCTGTTTGTAACTGTATCCAAGGGTTCAATCCCCGGAATAT

        950
ACAGCAGTGGGATCAGAGAGTCTGGGCAGGTGGGTGTATAAGGAGGACGCGGCTTAGCTGCAGTGGAGATGGTTTTACAAGGATGAAGAACATGAAGC

    1050                                         1100
TGCCAGAAACTACGATGGCGATTGTCGACCGCAGTATTGGTGTGAAAGAATGTGAGAAGAGGTGCCTTAGCGATTGTAATTGTACTGCTTTTGCAAAT

        1150                                         1200
GCAAATGCGGATATCCGGAATGGTGGGACGGGTTGTGTGATTTGGACCGGACGGCTTGACGATATGCGGAATTACGTTGCTCACGGTCAAGATCTTTATGTCAG

        1250
ATTGGCTGTTGCTGACCTTGTTTAGCTCTTTCTCTTAAAATAAAGCACGGATCC-3'
```

and any microorganism transformed by a transfer vector containing any fragment of said DNA base sequence which is unique to said

DNA base sequence and any functional equivalent of said DNA base sequence or fragment thereof.

13.  A fusion protein comprising an S-locus specific glycoprotein from a self-incompatible plant, a unique fragment of said glycoprotein, or any functional equivalent thereof and another protein or fragment thereof.

14.  A fusion protein of Claim 13, wherein said S-locus specific glycoprotein has the following amino acid sequence.

                                              50
NH- Gly Ser Val Val Leu Ile Leu Phe Cys Pro Ala Phe Ser Ile Asn Thr Leu Ser Ser

                                         100
Thr Glu Ser Leu Arg Ile Ser Ser Asn Arg Thr Leu Val Ser Pro Gly Asn Asn Phe Glu

                                    150
Leu Gly Phe Phe Arg Thr Asn Ser Ser Ser Arg Trp Tyr Leu Gly Ile Trp Tyr Lys Lys

                               200
Leu Leu Asp Arg Thr Tyr Val Trp Val Ala Asn Arg Asp Asn Pro Leu Ser Asn Ala Ile

          250
Gly Thr Leu Lys Ile Ser Gly Asn Asn Leu Val Leu Leu Gly His Thr Asn Lys Ser Val

                                         350
Trp Ser Thr Asn Leu Thr Arg Gly Asn Glu Arg Leu Arg Trp Trp Gln Thr Phe Ser Leu

Met Gly Thr Ser -COOH

15.  A fusion protein that is capable of reacting with antiserum to S-locus specific glycoprotein from a self-incompatible plant.

16. A method for cloning a cDNA sequence coding for at least a portion of an S-locus specific glycoprotein of the plant family Brassicaceae comprising:

(a) creating a cDNA library with clones containing cDNA to polyA+ RNA from incompatible stigmas or incompatible styles;

(b) hybridizing DNA from said clones with incompatible stigma or incompatible style cDNA probes and seedling derived cDNA probes;

(c) selecting a clone subset having DNA which hybridizes with said stigma or style cDNA probes and does not hybridize with said seedling cDNA probes;

(d) preparing and amplifying transfer vector DNA containing DNA from said clone subset; and

(e) digesting said transfer vector DNA to separate said transfer vector DNA from said DNA from said clone subset.

17. A method for cloning a cDNA sequence coding for at least a portion of an S-locus specific glycoprotein of the plant family Brassicaceae comprising:

(a) creating a cDNA library with clones containing cDNA to polyA+ RNA from incompatible stigmas;

(b) hybridizing DNA from said clones with stigma-derived cDNA probes and seedling derived cDNA probes;

(c) selecting a clone subset having DNA which hybridizes with said stigma-derived cDNA probes and does not hybridize with said seedling derived cDNA probes;

(d) preparing and amplifying transfer vector DNA containing DNA from said clone subset; and

(e) digesting said transfer vector DNA to separate said transfer vector DNA from said DNA from said clone subset.

0212385

FIGURE 1

## FIG.3a

L

BamHI          XhoI        BamHI

S

## FIG.3b

ClaI

PO    LacZ

pWR590
~ 4.7 kb

Ap

- SacI
- EcoRI
- SacI
- SmaI
- BamHI
- XbaI
- SalI
- PstI
- HindIII

## FIG.3c

pWR590/L

582 583     Sac I                    Bam HI
GGC AAC CGG GCG AGC TCG AAT TCG AGC TCG CCC GGG GAT CCG TCG TCT TGA
Gly Asn Arg Ala Ser Ser Asn Ser Ser Ser Pro Gly Asp Pro Ser Ser Stop

pWR590-1/L

582 583     Sac I                    Bam HI
GGC AAC CCG GGC GAG CTC GAA TTC GAG CTC GCC CGG GGA TCC GTC GTC (357 nucs) TGA
Gly Asn Pro Gly Glu Leu Glu Phe Glu Leu Ala Arg Gly Ser Val Val (119 aas) Stop

pWR590-2/L

598 599     Sac I                    Bam HI
GAT CGC GGG CGA GCT CGA ATT CGA GCT CGC CCG GGG ATC CGT CGT CTT (54 nucs) TAG
Asp Arg Gly Arg Ala Arg Ile Arg Ala Arg Pro Gly Ile Arg Arg Leu (18 aas) Stop

0212385

```
         BamHI                                                        50
5'-   GGA  TCC  GTC  GTC  TTG  ATT  CTA  TTT  TGT  CCT  GCC  TTT  TCG  ATC  AAC  ACT  TTG  TCG  TCT
      Gly  Ser  Val  Val  Leu  Ile  Leu  Phe  Cys  Pro  Ala  Phe  Ser  Ile  Asn  Thr  Leu  Ser  Ser


                                                       100
ACA  GAA  TCT  CTT  AGA  ATC  TCA  AGC  AAC  AGA  ACA  CTT  GTA  TCT  CCA  GGT  AAT  AAC  TTC  GAA
Thr  Glu  Ser  Leu  Arg  Ile  Ser  Ser  Asn  Arg  Thr  Leu  Val  Ser  Pro  Gly  Asn  Asn  Phe  Glu


                                       150
CTC  GGC  TTC  TTC  CGA  ACC  AAC  TCA  AGT  TCT  CGT  TGG  TAT  CTC  GGG  ATA  TGG  TAC  AAG  AAA
Leu  Gly  Phe  Phe  Arg  Thr  Asn  Ser  Ser  Ser  Arg  Trp  Tyr  Leu  Gly  Ile  Trp  Tyr  Lys  Lys


                              200
TTG  CTC  GAC  AGA  ACC  TAT  GTA  TGG  GTT  GCC  AAC  AGA  GAT  AAC  CCA  CTC  TCC  AAT  GCC  ATT
Leu  Leu  Asp  Arg  Thr  Tyr  Val  Trp  Val  Ala  Asn  Arg  Asp  Asn  Pro  Leu  Ser  Asn  Ala  Ile


                    250
GGA  ACC  CTC  AAA  ATC  TCA  GGC  AAT  AAT  CTT  GTC  CTC  CTT  GGT  CAC  ACC  AAT  AAA  TCT  GTT
Gly  Thr  Leu  Lys  Ile  Ser  Gly  Asn  Asn  Leu  Val  Leu  Leu  Gly  His  Thr  Asn  Lys  Ser  Val


     SalI                                                       350
TGG  TCG  ACG  AAT  CTT  ACT  AGA  GGA  AAT  GAG  AGA  CTT  CGG  TGG  TGG  CAG  ACG  TTC  TCT  CTA
Trp  Ser  Thr  Asn  Leu  Thr  Arg  Gly  Asn  Glu  Arg  Leu  Arg  Trp  Trp  Gln  Thr  Phe  Ser  Leu


                              400
ATG  GAA  ACT  TCG  TGA  TGCGAGACTCCAGTAACAACGACGCAAGTGAATACTTGTGGCAAAGTTTCGATTACCCTACGGATACTTTGCT
Met  Glu  Thr  Ser


450                                          500
TCCAGAGATGAAACTGGGTTACGACCTCAAAACAGGGGTTGAACAGGTTCCTTACATCATGGAGAAGTTCAGATGATCCATCAAGCGGGGATTTCTCGT


    550                                      600
ACAAGCTCGAAACCCGAAGCCTTCCTGAGTTTTATCTATGGCATGGGATCTTTCCAATGCATCGGAGTGGTCCATGGAATGGAGTCCGATTTAGTGGC


    650                                      700
ATACCAGAGGACCAAAAGCTGAGTTACATGGTGTACAATTTCACAGAGAATAGTGAAGAGGTCGCTTATACATTCCGAATGACCAACAACAGCATCTA


   XhoI  750                                 800
CTCGAGATTGACACTAAGTTCCGAAGGTATTTTCAGCGACTTACGTGGAATCCGTCAATAGGGATATGGACAGGTTCTGGTCTTCTCCAGTGGACCCC


       850                                   900
CAGTGCGATACATACATAATGTGCGGGCCTTACGCTTACTGTGGCGTGAACACATCACCTGTTTGTAACTGTATCCAAGGGTTCAATCCCCGGAATAT


      950                                              PstI
ACAGCAGTGGGATCAGAGAGTCTGGGCAGGTGGGTGTATAAGGAGGACGCGGCTTAGCTGCAGTGGAGATGGTTTTACAAGGATGAAGAACATGAAGC


       1,050          SalI                             1,100
TGCCAGAAACTACGATGGCGATTGTCGACCGCAGTATTGGTGTGAAAGAATGTGAGAAGAGGTGCCTTAGCGATTGTAATTGTACTGCTTTTGCAAAT


       1,150                              1,200          BglII
GCGGATATCCGGAATGGTGGGACGGGTTGTGTGATTTGGACCGGACGGCTTGACGATATGCGGAATTACGTTGCTCACGGTCAAGATCTTTATGTCAG


      1,250                            BamHI
ATTGGCTGTTGCTGACCTTGTTTAGCTCTTTCTCTTAAAAATAAAGCACGGATCC-3'
```

<p style="text-align:center">FIG.2</p>